# EUROPÄISCHE PATENTSCHRIFT

(11) **EP 3 934 613 B1**
(45) Veröffentlichungstag und Bekanntmachung des Hinweises auf die Patenterteilung: **01.11.2023**
(21) Anmeldenummer: 20705982.5
(22) Anmeldetag: 21.02.2020
(51) Int. Cl.: A61K 8/60, A61Q 5/12

(54) **RHAMNOLIPIDE ALS DEPOSITIONSHILFE**
RHAMNOLIPIDS AS DEPOSITION AID
RHAMNOLIPIDES EN TANT QU' AUXILIAIRES DE DÉPÔT

(30) Priorität: 07.03.2019 EP 19161191
(43) Veröffentlichungstag der Anmeldung: 12.01.2022
(73) Patentinhaber: Evonik Operations GmbH, 45128 Essen (DE)
(72) Erfinder: KLEINEN, Jochen, 52525 Heinsberg (DE); BRANDT, Kathrin Daniela, 40476 Düsseldorf (DE); SCHULZ, Meike, 45279 Essen (DE); MUSS, Alina, 45359 Essen (DE)
(74) Vertreter: Evonik Patent Association
(86) Internationale Anmeldenummer: PCT/EP2020/054623
(87) Internationale Veröffentlichungsnummer: WO 2020/178048

(56) Entgegenhaltungen:
- WO-A1-2018/145966
- CN-A- 108 901 546
- JP-A- 2002 105 854
- US-A1- 2011 318 294
- CHEN JIANWEI ET AL: "Potential applications of biosurfactant rhamnolipids in agriculture and biomedicine", APPLIED MICROBIOLOGY AND BIOTECHNOLOGY, SPRINGER, DE, Bd. 101, Nr. 23, 10. Oktober 2017 (2017-10-10), Seiten 8309-8319, XP036366895, ISSN: 0175-7598, DOI: 10.1007/S00253-017-8554-4 [gefunden am 2017-10-10]

## Beschreibung

### Gebiet der Erfindung

Gegenstand der Erfindung ist die Verwendung von Rhamnolipiden zur Deposition mindestens einer Substanz aus einem Medium auf einer Oberfläche, dadurch gekennzeichnet, dass die Substanz ausgewählt ist aus der Gruppe umfassend, Guar-Quats, Siloxan-Quats und Cellulose-Quats.

### Stand der Technik

Aufgabe der Erfindung war es, Substanzen zu identifizieren, die die Deposition mindestens einer Substanz aus einem Medium auf einer Oberfläche, insbesondere Haut und Haaren, begünstigen.

In vielen Anwendungen werden Substanzen aus einer Formulierung heraus auf einer Oberfläche depositioniert, um der Oberfläche eine gewünschte Eigenschaft zu verleihen.

Die Deposition von Konditioniermitteln wie beispielsweise Polyquats auf Haut und Haaren führt beispielsweise zu einer verbesserten Sensorik, weshalb Shampooformulierungen oftmals solche Konditioniermittel enthalten.

In den allermeisten Anwendungen werden allerdings nicht die theoretisch verfügbaren Gesamtmengen an zu depositionierender Substanz depositioniert. Eine Maximierung der Deposition an Substanz ist anzustreben, um ressourcenschonend arbeiten zu können.

Als Stand der Technik, offenbart US 2011/318294 A1 die Verwendung von einer Mischung aus alkoxylierten und nicht-alkoxylierten Phosphatestern zur Deposition von polykationischen Verbindungen zur Verbesserung der haarkonditionierenden Eigenschaften.

### Beschreibung der Erfindung

Überraschenderweise wurde gefunden, dass bei Einsatz von Rhamnolipiden eine hervorragende Deposition erreicht wird.

Gegenstand der vorliegenden Erfindung ist daher die Verwendung von Rhamnolipiden zur Deposition mindestens einer Substanz aus einem Medium auf einer Oberfläche, dadurch gekennzeichnet, dass die Substanz ausgewählt ist aus der Gruppe umfassend, Guar-Quats, Siloxan-Quats und Cellulose-Quats.

Ein Vorteil der Erfindung ist es, dass bei der erfindungsgemäßen Verwendung eine sehr gute Sensorik erzielt wird.

Ein weiterer Vorteil der Erfindung ist, dass bei der erfindungsgemäßen Verwendung Eigenschaften wie Kämmbarkeit, Weichheit, Formbarkeit, Glanz, Handhabbarkeit und die Entwirrbarkeit von Haaren verbessert werden können.

Noch ein Vorteil der Erfindung ist es, dass bei der erfindungsgemäßen Verwendung die Reizwirkung für Haut und Schleimhäute gering ist.

Ein weiterer Vorteil der Erfindung ist es, dass bei der erfindungsgemäßen Verwendung komplett auf nachwachsenden Rohstoffen basierende Zusammensetzungen verwendet werden können. Noch ein Vorteil der vorliegenden Erfindung ist, dass man Formulierungen bereitstellen kann, die siliconfrei sind, aber dennoch gute konditionierende Eigenschaften auf Haut und Haare aufweisen. Noch ein Vorteil der Erfindung ist es, dass bei der erfindungsgemäßen Verwendung ein hohes Schaumvolumen erreicht werden kann.

Ein weiterer Vorteil ist, dass das Schaumvolumen der erfindungsgemäßen Formulierung auch in Gegenwart von Sebum auf Haut und Haaren nicht beeinträchtigt wird.

Noch ein Vorteil der Erfindung ist es, dass bei der erfindungsgemäßen Verwendung eine besonders gute Schaumcremigkeit erreicht werden kann.

Ein weiterer Vorteil der Erfindung ist es, dass sich bei der erfindungsgemäßen Verwendung gut verdickbare Formulierungen ergeben.

Als zusätzlicher Vorteil kann die herausragende Kompatibilität des Rhamnolipid genannt werden. Noch ein Vorteil der vorliegenden Erfindung ist es, dass sie die Kammkräfte am nassen und am trockenen Haar reduziert.

Die erfindungsgemäße Verwendung ist, sofern die Oberfläche die eines lebenden Körpers ist, eine nicht-therapeutische Verwendung. Insbesondere stellt in diesem Zusammenhang die erfindungsgemäße Verwendung eine kosmetische Verwendung dar.

Alle angegebenen Prozent (%) sind, wenn nicht anders angegeben, Massenprozent.

Gegenstand der vorliegenden Erfindung ist die Verwendung von Rhamnolipiden zur Deposition mindestens einer Substanz aus einem Medium auf einer Oberfläche, dadurch gekennzeichnet, dass die Substanz ausgewählt ist aus der Gruppe umfassend Guar-Quats, Siloxan-Quats und Cellulose-Quats.

Unter dem Begriff "Rhamnolipid" im Zusammenhang mit der vorliegenden Erfindung werden Rhamnolipide, deren protonierten Formen sowie insbesondere deren Salze mitumfasst.

Unter dem Begriff "Rhamnolipid" im Zusammenhang mit der vorliegenden Erfindung werden insbesondere Mischungen aus Verbindungen der allgemeinen Formel (I) und deren Salze verstanden, 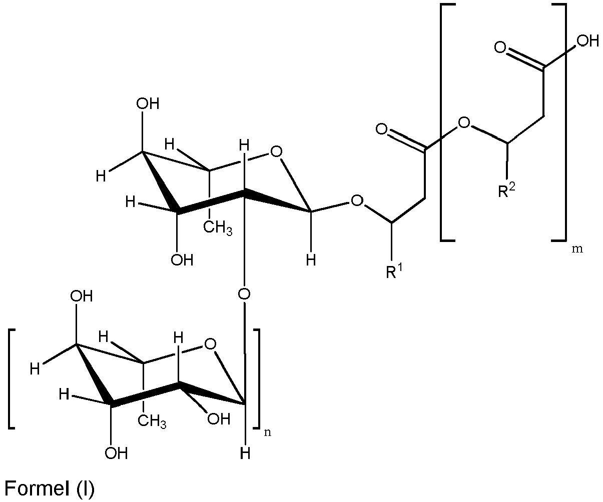 wobei
m = 2, 1 oder 0,
n = 1 oder 0,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undecenyl und Tridecenyl und (CH₂)ₒ-CH₃ mit o = 1 bis 23, bevorzugt 4 bis 12.

Falls n = 1 ist die glykosidische Bindung zwischen den zwei Rhamnoseeinheiten bevorzugt in der α-Konfiguration. Die optisch aktiven Kohlenstoffatome der Fettsäuren liegen bevorzugt als R-Enantiomere vor (z.B. (R)-3-{(R)-3-[2-O-(α-L-rhamnopyranosyl)-α-L-rhamnopyranosyl]oxydecanoyl}oxydecanoate).

Unter dem Begriff "di-Rhamnolipid" im Zusammenhang mit der vorliegenden Erfindung werden Verbindungen der allgemeinen Formel (I) oder deren Salze verstanden, bei denen n = 1.

Unter dem Begriff "mono-Rhamnolipid" im Zusammenhang mit der vorliegenden Erfindung werden Verbindungen der allgemeinen Formel (I) oder deren Salze verstanden, bei denen n = 0. Distinkte Rhamnolipide werden gemäß folgender Nomenklatur abgekürzt:
Unter "diRL-CXCY" werden di-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Unter "monoRL-CXCY" werden mono-Rhamnolipide der allgemeinen Formel (I) verstanden, bei denen einer der Reste R¹ und R² = (CH₂)ₒ-CH₃ mit o = X-4 und der verbleibende Rest R¹ oder R² = (CH₂)ₒ-CH₃ mit o = Y-4.

Die verwendete Nomenklatur unterscheidet somit nicht zwischen "CXCY" und "CYCX".

Für Rhamnolipide mit m=0 wird entsprechend monoRL-CX bzw. diRL-CX verwendet.

Ist einer der oben genannten Indices X und/oder Y mit ":Z" versehen, so bedeutet dies, dass der jeweilige Rest R¹ und/oder R² = ein unverzweigter, unsubstituierter Kohlenwasserstoffrest mit X-3 bzw. Y-3 Kohlenstoffatomen aufweisend Z Doppelbindungen darstellt.

Zur Bestimmung des Gehaltes an Rhamnolipiden im Zusammenhang mit der vorliegenden Erfindung wird lediglich die Masse des Rhamnolipid-Anions, somit "allgemeinen Formel (I) abzüglich ein Wasserstoff" berücksichtigt.

Zur Bestimmung des Gehaltes an Rhamnolipiden im Zusammenhang mit der vorliegenden Erfindung werden alle Rhamnolipide durch Ansäuern in die protonierte Form (vgl. allgemeine Formel (I)) überführt und mittels HPLC quantifiziert.

Es ist erfindungsgemäß bevorzugt, dass die verwendeten Rhamnolipide 51 Gew.-% bis 95 Gew.-%, bevorzugt 70 Gew.-% bis 90 Gew.-%, besonders bevorzugt 75 Gew.-% bis 85 Gew.-%, diRLC10C10 enthalten, wobei sich die Gewichtsprozente auf die Summe aller verwendeten Rhamnolipide beziehen.

Es ist erfindungsgemäß bevorzugt, dass die verwendeten Rhamnolipide 0,5 Gew.-% bis 9 Gew.-%, bevorzugt 0,5 Gew.-% bis 3 Gew.-%, besonders bevorzugt 0,5 Gew.-% bis 2 Gew.-%, monoRL-C10C10 enthalten, wobei sich die Gewichtsprozente auf die Summe aller verwendeten Rhamnolipide beziehen.

Eine bevorzugte erfindungsgemäße Verwendung ist dadurch gekennzeichnet, dass das Gewichtsverhältnis von allen verwendeten di-Rhamnolipiden zu allen verwendeten mono-Rhamnolipiden größer 51:49, insbesondere größer 91:9, bevorzugt größer 97:3, besonders bevorzugt größer 98:2, ist.

Es ist erfindungsgemäß bevorzugt, dass die verwendeten Rhamnolipide 0,5 bis 25 Gew.-%, bevorzugt 5 Gew.-% bis 15 Gew.-%, besonders bevorzugt 7 Gew.-% bis 12 Gew.-%, diRL-C10C12 enthalten, wobei sich die Gewichtsprozente auf die Summe aller verwendeten Rhamnolipide beziehen.

Es ist erfindungsgemäß bevorzugt, dass die verwendeten Rhamnolipide 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und/oder, bevorzugt und, 0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1, enthalten, wobei sich die Gewichtsprozente auf die Summe aller verwendeten Rhamnolipide beziehen.

Eine besonders bevorzugte erfindungsgemäße Verwendung ist dadurch gekennzeichnet, dass die verwendeten Rhamnolipide
0,5 Gew.-% bis 15 Gew.-%, bevorzugt 3 Gew.-% bis 12 Gew.-%, besonders bevorzugt 5 Gew.-% bis 10 Gew.-%, diRL-C10C12:1,
0,5 bis 25 Gew.-%, bevorzugt 5 Gew.-% bis 15 Gew.-%, besonders bevorzugt 7 Gew.-% bis 12 Gew.-%, diRL-C10C12,
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12 und
0,1 Gew.-% bis 5 Gew.-%, bevorzugt 0,5 Gew.-% bis 3 Gew.-%, besonders bevorzugt 0,5 Gew.-% bis 2 Gew.-%, monoRL-C10C12:1,
enthalten, wobei sich die Gewichtsprozente auf die Summe aller verwendeten Rhamnolipide beziehen.

Es ist überdies hinaus bevorzugt, wenn die erfindungsgemäß verwendeten Rhamnolipide nur kleinen Mengen der Formel monoRL-CX bzw. diRL-CX in enthalten. Insbesondere enthalten die erfindungsgemäß verwendeten Rhamonlipide bevorzugt
0 Gew.-% bis 5 Gew.-%, bevorzugt 0 Gew.-% bis 3 Gew.-%, besonders bevorzugt 0,1 Gew.-% bis 1 Gew.-%, diRLC10, wobei sich die Gewichtsprozente auf die Summe aller verwendeten Rhamnolipide beziehen.

Eine erfindungsgemäß bevorzugte Verwendung ist dadurch gekennzeichnet, dass die Substanz ausgewählt ist aus der Gruppe umfassend, bevorzugt bestehend aus, Guar-Quats, Siloxan-Quats und Cellulose-Quats.

Eine erfindungsgemäß bevorzugte Verwendung ist dadurch gekennzeichnet, dass das Medium ausgewählt ist aus der Gruppe umfassend, bevorzugt bestehend aus, Tensidmischungen, Emulsionen und Mikroemulsionen, insbesondere O/W-Emulsionen, W/O-Emulsionen, Wasser-in-Silicon-Emulsionen, Silicon-in-Wasser-Emulsionen, O/W-Mikroemulsionen, W/O-Mikroemulsionen, Wasser-in-Silicon-Mikroemulsionen, Silicon-in-Wasser-Mikroemulsionen sowie Dispersionen.

Eine erfindungsgemäß bevorzugte Verwendung ist dadurch gekennzeichnet, dass das Medium ausgewählt ist aus der Gruppe umfassend, bevorzugt bestehend aus, kosmetische Formulierungen, insbesondere Shampoos, Konditioner, Duschgele, Badeseifen, Badeöle, Gele, Schäume und Puder.

Die im Rahmen der bevorzugten erfindungsgemäßen Verwendung eingesetzten kosmetischen Formulierungen können mindestens eine zusätzliche Komponente enthalten, ausgewählt aus der Gruppe der
Emollients,
Emulgatoren,
Verdicker/Viskositätsregler/Stabilisatoren,
UV-Lichtschutzfilter,
Antioxidantien,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Farbstoffe,
kosmetische Wirkstoffe,
Pflegeadditive,
Überfettungsmittel,
Lösungsmittel.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.

Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.

Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Eine erfindungsgemäß bevorzugte Verwendung ist dadurch gekennzeichnet, dass die Rhamnolipide in dem Medium in einer Konzentration von 0,1 Gew.-% bis 30 Gew.-%, bevorzugt von 0,5 Gew.-% bis 25 Gew.-%, besonders bevorzugt von 1,0 Gew.-% bis 12 Gew.-%, enthalten sind, wobei sich die Gewichtsprozente auf das Gesamtmedium beziehen.

Eine erfindungsgemäß bevorzugte Verwendung ist dadurch gekennzeichnet, dass die Substanz in dem Medium in einer Konzentration von 0,05 Gew.-% bis 5 Gew.-%, bevorzugt von 0,1 Gew.-% bis 3 Gew.-%, besonders bevorzugt von 0,15 Gew.-% bis 1,5 Gew.-%, enthalten ist, wobei sich die Gewichtsprozente auf das Gesamtmedium beziehen.

Eine erfindungsgemäß bevorzugte Verwendung ist dadurch gekennzeichnet, dass der pH-Wert des Mediums bei 25 °C in einem Bereich von 3,5 bis 9,0 bevorzugt von 3,8 bis 7,0 besonders bevorzugt von 4,4 bis 6,6 liegt.

Der "pH-Wert" im Zusammenhang mit der vorliegenden Erfindung ist definiert als der Wert, welcher für die entsprechende Zusammensetzung bei 25 °C nach fünf Minuten Rühren mit einer gemäß ISO 4319 (1977) kalibrierten pH-Elektrode gemessen wird.

Eine erfindungsgemäß bevorzugte, nicht-therapeutische Verwendung ist dadurch gekennzeichnet, dass die Oberfläche ausgewählt ist aus Haut und Haaren, insbesondere Haaren.

### Beispiele:

### Beispiel 1: Deposition von quaternisiertem Guar auf Haaren

Dieses Beispiel zeigt, dass der Austausch verschiedener Tenside (SLES und unterschiedliche Betaine) durch Rhamnolipide die Menge an depositioniertem, quaternisierten Guar signifikant erhöht. Als Rhamnolipide wurde im Folgenden RHEANCE^{®} One (Evonik Industries, 50%ig, INCI: Glycolipids) verwendet. Die Formulierungsbestandteile sind in den folgenden Zusammensetzungen in Form der allgemein anerkannten INCI-Nomenklatur unter Verwendung der englischen Begriffe benannt. Alle Konzentrationen in den Anwendungsbeispielen 1a-e sind in Aktivgehalt angegeben. Das Ausmaß der Deposition des Guars wurde zum einen durch die Messung des Zeta-Potentials (Oberflächen-Potentials) ermittelt. Dazu wird das Zeta-Potential auf der Haarfaser gemessen. Der Zusammenhang zwischen depositionierter Menge kationischem Polymer und dem Zeta-Potential with in J. Jachowicz, M. Berthiaume, and M. Garcia Colloid & Polymer Sci 263:847-858 (1985) beschrieben, ein weniger stark negatives Zeta-Potential entspricht einer höheren Deposition.

Das Zeta-Potential der Haare wurde mit einem Fiber Potential Analyzer (FPA, Firma emtec, Leipzig, Deutschland) gemessen. Das Zeta-Potential der Haarsträhnen wurde vor der Behandlung bestimmt, da der Schädigungsgrad einen Einfluss auf das Zeta-Potential hat und innerhalb einer Reihe nur Haare gleicher Schädigung / Zeta-Potential verwendet wurde. Dabei steht ein stärker negatives Zeta-Potential, kontraintuitiv, für ein weniger stark geschädigtes Haar. In der Folge kann durch die Deposition eines kationischen Polymers auf einem weniger stark geschädigten Haar ein stärker positives Zeta-Potential erzielt werden.

Zwei Qualitäten an Haaren konnten durch die Vormessungen identifiziert werden, zum einen Haare mit -31,0 mV und stärker geschädigte mit -25,0 mV Zeta-Potential.

Zur Messung wurden die Haarsträhnen (Kehrling) mit den entsprechenden Formulierungen entsprechend wie zur Bestimmung der sensorischen Eigenschaften behandelt (Paneltest, siehe unten).

Um das Zeta-Potential der Haare zu messen wurde eine Haartresse (4 g) in die Messkammer des FPAs gegeben und mit Leitungswasser durchströmt. 1 g NaCl wurden in 2 Litern Wasser gelöst, um konstante Wasserqualitäten und Leitfähigkeitswerte zu erzielen, dieses Wasser wurde zur Messung benutzt. Die Strähnen wurden vor der Messung in das Wasser (1 g NaCl / 2 L) getaucht, um die Haare zu benetzen. Es wurden je Probe jeweils 10 Messungen im werksseitig eingestellten Auto-Modus durchgeführt und anschließend der Mittelwert gebildet. Alle genannten Werte sind Mittelwerte, die Schwankung der Einzelwerte betrug maximal 0,5 mV nach oben und 0,5 mV nach unten, die Reproduzierbarkeit kann ebenfalls mit 0,5 mV angegeben werden.

Die Formulierungen enthielten jeweils 0,5 % Guar und 12 % Tensid. Als Tenside wurden Sodium Laureth Sulfate (SLES), Cocamidopropyl Betaine (CAPB), Cocobetaine (COB) und Glycolipides (RL) verwendet.

Sind positive Messwerte erhalten worden, so sind diese vor dem Zahlenwert aufgeführt, um eine bessere Unterscheidbarkeit der Messwerte zu erreichen.

### Beispiel 1 a: Binäre Mischung aus Natrium Laurylether Sulfat (SLES) und Rhamnolipid (RL) (Angaben nach Aktivgehalt)

| SLES / % | RL / % | Guar / % | Zeta-Potential Haare / mV | Bemerkung |
|---|---|---|---|---|
| - | - | - | -25,0 | Haare unbehandelt |
| 12 | - | 0,5 | -23,3 | |
| 9 | 3 | 0,5 | -11,2 | |
| 6 | 6 | 0,5 | -3,6 | |
| | 12 | 0,5 | +2,6 | |
| - | - | 0,5 | +3,0 | nur Guar, maximal erreichbarer Wert |

### Beispiel 1 b: Binäre Mischung 9 % SLES bzw. 9 % RL; 3 % Cocamidopropyl Betaine (CAPB) bzw. 3 % Cocobetain (COB) (Angaben nach Aktivgehalt)

| 9% | 3% | Guar / % | Zeta-Potential Haare / mV | Bemerkung |
|---|---|---|---|---|
| - | - | - | -31,0 | Haare unbehandelt |
| SLES | CAPB | 0,5 | -6,2 | |
| SLES | CAPB | 0,5 | -6,4 | Überprüfung Reproduzierkeit |
| RL | CAPB | 0,5 | +6,6 | |
| SLES | COB | 0,5 | -9,9 | |
| - | - | 0,5 | +8,5 | nur Guar, maximal erreichbarer Wert |

### Beispiel 1 c: Binäre Mischung 6 % SLES bzw. 6 % RL; 6 % CAPB bzw. 6 % COB (Angaben nach Aktivgehalt)

| 6 % | 6 % | Guar / % | Zeta-Potential Haare / mV | Bemerkung |
|---|---|---|---|---|
| - | - | - | -25,0 | Haare unbehandelt |
| SLES | CAPB | 0,5 | -15,7 | |
| RL | CAPB | 0,5 | -11,8 | |
| SLES | COB | 0,5 | -18,0 | |
| - | - | 0,5 | +3,0 | nur Guar, maximal erreichbarer Wert |

### Beispiel 1 d: Teilersatz in SLES COB Formulierungen (Angaben nach Aktivgehalt)

| SLES / % | COB / % | RL / % | Guar / % | Zeta-Potential Haare / mV | Bemerkung |
|---|---|---|---|---|---|
| - | - | - | - | -25,0 | Haare unbehandelt |
| 6 | 6 | - | 0,5 | -18,0 | |
| 6 | 3 | 3 | 0,5 | -7,0 | |
| 9 | 3 | - | 0,5 | -9,9 | |
| 9 | 1,5 | 1,5 | 0,5 | -7,9 | |
| - | - | - | 0,5 | +3,0 | nur Guar, maximal erreichbarer Wert |

### Beispiel 1 e: Einzeltenside, 12 % Tenside (Angaben nach Aktivgehalt)

| 12 % | Guar / % | Zeta-Potential Haare / mV | Bemerkung |
|---|---|---|---|
| - | - | -25,0 | Haare unbehandelt |
| SLES | 0,5 | -23,3 | |
| RL | 0,5 | +2,6 | |
| CAPB | 0,5 | -8,7 | |
| - | 0,5 | +3,0 | nur Guar, maximal erreichbarer Wert |

Die Ergebnisse der Messungen aus Beispielen 1 a bis 1 e zeigen jeweils, dass die Verwendung von Rhamnolipid in den Formulierungen ein weniger negatives Zeta-Potential und somit eine höhere Deposition des kationischen Polymers (Guar-Quat) zur Folge hat.

Beispiel 1 f:

Ebenso konnte das Ausmaß der Deposition des Guars durch die die verbesserte Sensorik (Paneltest), indem verschiedene Formulierungen auf Haarsträhnen aufgebracht und anschließend sensorisch beurteilt wurden, gezeigt werden. Die Formulierungsbestandteile sind in den folgenden Zusammensetzungen in Form der allgemein anerkannten INCI-Nomenklatur unter Verwendung der englischen Begriffe benannt. Alle Konzentrationen im Anwendungsbeispiel 1f sind in Gewichtsprozent angegeben.

Für die Beurteilung durch das sensorische Panel wurden drei einfache kosmetische Shampoo-Formulierungen hergestellt, die erfindungsgemäßen Beispiele A, B und C, die Rhamnolipid in unterschiedlichen Konzentrationen enthalten (siehe Tab. 1).

**Tab. 1: Shampooformulierungen zur Austestung der deponierenden Eigenschaften; Angaben in Gewichtsprozent**

| **Formulierungsbeispiele** | **A** | **B** | **C** |
|---|---|---|---|
| RHEANCE One, 50%-ig, Evonik (INCI: Glycolipids) | 24,0% | 12,0% | 6,0% |
| Texapon^{®} NSO, 28%-ig, Cognis (INCI: Sodium Laureth Sulfate) | -- | 21,4% | 32,1% |
| Jaguar^{®} C-162, 100%-ig, Solvay | 0,5% | 0,5% | 0,5% |
| (INCI: Hydroxypropyl Guar Hydroxypropyltrimonium Chloride) | | | |
| Wasser, demineralisiert | ad 100,0% | | |
| Zitronensäure | ad. pH 6,0 | | |

### Versuchsbedingungen für den sensorischen Haarsträhnentest:

Für den sensorischen Haarsträhnentest werden europäisches Haar der Fa. Kerling (vorgefertigte Haarbündel, 18 cm lang, 4 g; standardisiert vorgeschädigt durch Bleichen) sowie gesägte Plastikkämme (17 cm lang) mit einer feinen (ca. 9,5 Zähne/cm) und einer groben (ca. 4 Zähne/cm) Seite verwendet.

Die Haarsträhnen werden für den sensorischen Haarsträhnentest wie folgt mit den in Tab. 1 beschriebenen Shampoo-Formulierungen behandelt:

### Standardisierte Behandlung von vorgeschädigten Haarsträhnen mit Shampoo-Formulierungen:

Die Haarsträhnen werden unter fließendem, warmem Wasser benetzt. Das überschüssige Wasser wird leicht von Hand ausgedrückt, dann wird das Shampoo aufgebracht und sanft im Haar eingearbeitet (0.5 g Formulierung pro Haarsträhne (2 g)). Nach einer Verweilzeit von 1 min wird das Haar für 1 min unter fließendem Wasser ausgespült.

Anschließend erfolgt die sensorische Beurteilung der Haarsträhnen durch vier Testpersonen unter standardisierten Bedingungen bei 50% Luftfeuchtigkeit und 25 °C.

Die Beurteilung der Haarsträhnen durch das Panel erfolgt wie folgt:

### Beurteilung der Haarsträhnen durch das sensorische Panel:

Die Testkriterien sind: Entwirrbarkeit, Nasskämmbarkeit und Nassgriff der Haare. Die sensorischen Bewertungen erfolgen nach Noten, die auf einer Skala von 1 bis 5 vergeben werden, wobei 1 die schlechteste und 5 die beste Bewertung ist. Die einzelnen Testkriterien erhalten jeweils eine eigene Bewertung. Jeder Panel-Teilnehmer bewertet alle Kriterien für alle Testformulierungen. Jede Haarsträhne wird dabei nur einmal verwendet.

Details der Beurteilungskriterien sind in DE 103 27 871 beschrieben.

Die Ergebnisse der sensorischen Beurteilung durch das Panel der wie oben beschrieben durchgeführten Behandlung der Haarsträhnen mit der erfindungsgemäßen Formulierungen A, B und C sind in Tab. 2 dargestellt.

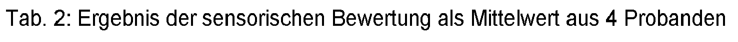

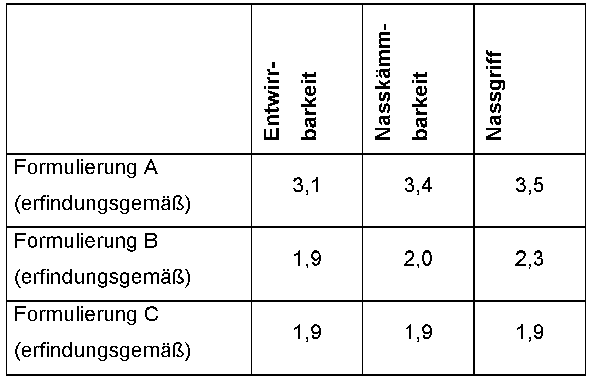

Die Ergebnisse des sensorischen Panels zeigen, dass mit steigendem Anteil an Rhamnolipid in der Formulierung für alle Testkriterien eine bessere Bewertung durch das sensorische Panel erzielt wird.

### Beispiel 2: Deposition von Polyquaternium-10 und Siliconquat auf Haaren

Dieses Beispiel zeigt, dass die Verwendung von Rhamnolipid an Stelle von Sodium Laureth Sulfate (SLES) die Menge an depositionierter, quaternisierter Hydroxyethyl-Cellulose signifikant erhöht. Dies wurde im Sensorik-Paneltest wie folgt bestätigt. Die Formulierungsbestandteile sind in den folgenden Zusammensetzungen in Form der allgemein anerkannten INCI-Nomenklatur unter Verwendung der englischen Begriffe benannt. Alle Konzentrationen im Anwendungsbeispiel 2 sind in Gewichtsprozent angegeben.

Für die Beurteilung durch das sensorische Panel wurden zwei einfache kosmetische Shampoo-Formulierungen hergestellt, das erfindungsgemäße Beispiel D sowie das nicht erfindungsgemäße Beispiel E (siehe Tab. 3).

**Tab. 3: Shampooformulierungen zur Austestung der deponierenden Eigenschaften.**

| **Formulierungsbeispiele** | **D** | **E** |
|---|---|---|
| RHEANCE One, (INCI: Rhamnolipid) | 18,0 | -- |
| Texapon^{®} NSO, 28%-ig, Cognis (INCI: Sodium Laureth Sulfate) | -- | 32,1% |
| TEGO^{®} Betain F 50, 38%-ig, Evonik (INCI: Cocamidopropyl Betaine) | 7,9% | 7,9% |
| Polymer JR 400, 100%-ig, Amerchol (INCI: Polyquaternium-10) | 0,2% | 0,2% |
| ABIL^{®} ME 45, 30%-ig, Evonik | 3,3% | 3,3% |
| (INCI: Silicone Quaternium-22; Polyglyceryl-3 Caprate; Dipropylene Glycol; Cocamidopropyl Betaine) | | |
| Wasser, demineralisiert | ad 100,0% | |
| Zitronensäure | ad. pH 6,0 | |

Es gelten die in Beispiel 1 f beschriebenen Versuchsbedingungen für den sensorischen Haarsträhnentest.

Die vorgeschädigten Haarsträhnen werden dann wie ebenfalls in Beispiel 1 f beschrieben mit den Shampoo-Formulierungen aus Tab. 3 behandelt.

Die sensorische Beurteilung erfolgt unter standardisierten Bedingungen bei 50% Luftfeuchtigkeit und 25 °C wie in Beispiel 1 f beschrieben.

Die Testkriterien sind: Entwirrbarkeit, Nasskämmbarkeit, Nassgriff. Details der Beurteilung können Beispiel 1 f sowie DE 103 27 871 entnommen werden.

Die Ergebnisse der sensorischen Beurteilung durch das Panel der wie oben beschrieben durchgeführten Behandlung der Haarsträhnen mit der erfindungsgemäßen Formulierungen D und der nicht erfindungsgemäßen Vergleichsformulierung E sind in Tabelle 4 dargestellt.

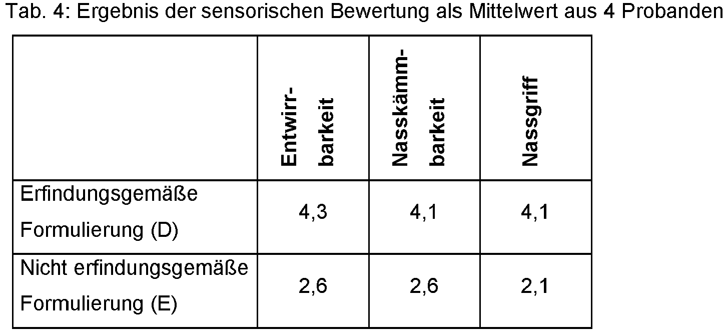

Die Ergebnisse des sensorischen Panels zeigen, dass die Verwendung von Rhamnolipid in der Formulierung (erfindungsgemäße Formulierung D) zu einer signifikant besseren Bewertung führt als die Verwendung von Sodium Laureth Sulfate (nicht erfindungsgemäße Formulierung E), was sich auf eine erhöhte Deposition des kationischen Polymers (Polyquaternium-10) im Falle der Verwendung von Rhamnolipid zurückführen lässt.

Die verbesserte Deposition des kationischen Polymers kann des Weiteren durch Messungen der Kämmkräfte auf Haaren gezeigt werden, wie das folgende Beispiel illustriert:

### Versuchsbedingungen der Kämmkraftmessungen

Messgerät: Diastron MTT 175
Messstrecke: 16,5 cm
Kämmgeschwindigkeit: 2000 mm/min
Verwendete Haarsträhnen: Länge = ca. 20 cm (Gesamtlänge - 18 cm freies Haar); Breite = 1,5 cm; Gewicht (trocken) = 3 g
Messbedingungen: T = 22°C.
Die Haarsträhnen werden mit einer Restfeuchte von 60%, bestimmt durch Gewichtsbestimmung, gemessen.

Für die Versuche wird europäisches, 4 h gebleichtes Haar der Fa. Kerling verwendet (vorgefertigte Flachtressen, 1,5 cm breit, 3 g).

### Durchführung der Kämmkraftmessungen

1. Die Haarsträhne wird 20 sec in einer Pufferlösung (Na-Citrat, pH = 6) eingetaucht.
2. Die Haarsträhne wird so lange von Hand vorgekämmt, bis keine Änderung des Kämmwiderstandes festzustellen.
3. Die Haarsträhne wird am Messgerät befestigt und die erste Kämmkraftmessung wird durchgeführt. Die Messung wird insgesamt 10-mal wiederholt. Vor jeder weiteren Messung wird die Haarsträhne durch zwei Sprühstöße aus einem Deo-Pumpzerstäuber mit der Pufferlösung angefeuchtet. Der Mittelwert der Kämmarbeit in % wird mithilfe der MTT175-Software bestimmt.

Zunächst werden alle Haarsträhnen unbehandelt gemessen. Anschließend werden die Haarsträhnen mit den Testformulierungen wie folgt behandelt:

### Behandlung der Haarsträhnen für die Kämmkraftmessungen

Pro Haarsträhne werden 0,5 g der jeweiligen Testformulierung verwendet (2 g Haar/0,5 g Lösung). Die Formulierung wird 30 sec. in das Haar einmassiert, dann für 1 min. ruhen gelassen und anschließend 30 sec unter fließendem Leitungswasser von 37°C abgespült. Anschließend wird die Haarsträhne ein weiteres Mal für 30 sec. shampooniert, 1 min. ruhen gelassen und schließlich 1 min unter fließendem Leitungswasser von 37°C ausgespült.

### Durchführung der Kämmkraftmessungen

Für die Durchführung der Kämmkraftmessung mit den Testformulierungen werden die Punkte 1-3 wiederholt. Pro Testformulierung werden jeweils 4 Haarsträhnen gemessen. Der Mittelwert der Kämmarbeit in % wird anschließend mithilfe der MTT175-Software bestimmt.

Mithilfe der beschrieben Methode wurden die Kämmkräfte für die Rhamnolipid-haltige Formulierung A (erfindungsgemäß) mit der Vergleichformulierung B (nicht erfindungsgemäß), die Sodium Laureth Sulfate anstelle von Rhamnolipid enthält, verglichen. Die Ergebnisse sind in Tab. 5 dargestellt.

**Tab. 5: Ergebnis der Kämmkraftmessungen als Mittelwert aus 4 Einzelwerten; dargestellt ist die Reduktion der Kämmarbeit in %**

| | Reduktion der Kämmarbeit [%] |
|---|---|
| Erfindungsgemäße Formulierung (D) | 49 (±7) |
| Nicht erfindungsgemäße Formulierung (E) | 18 (±12) |

Die Ergebnisse zeigen, dass die erfindungsgemäße Formulierung A eine ausgeprägtere Reduktion der Kämmkräfte aufweist als die Vergleichsformulierung B.

### Weitere Formulierungsbeispiele

* Formulierungen, die nicht Teil der Erfindung sind.

Die Formulierungsbestandteile sind in den folgenden Zusammensetzungen in Form der allgemein anerkannten INCI-Nomenklatur unter Verwendung der englischen Begriffe benannt. Alle Konzentrationen in den Anwendungsbeispielen sind in Aktivgehalt angegeben.

| Beispiel 3 | a | b | c | d | e | f | 9 | h | i |
|---|---|---|---|---|---|---|---|---|---|
| RHEANCE^{®} One (Glycolipids) | 2,5 | 2,5 | 2,5 | 2,5 | 2,5 | 2,5 | 2,5 | 2,5 | 2,5 |
| Isostearamide MIPA; Glyceryl Laurate | 1 | 1 | 1 | 1 | 1 | 1 | 1 | 1 | 1 |
| Sorbitan Sesquicaprylate | 0,5 | 0,5 | 0,5 | 0,5 | 0,5 | 0,5 | 0,5 | 0,5 | 0,5 |
| Cocamidopropyl Betaine | 5 | 5 | 5 | 5 | 5 | 5 | 5 | 5 | 5 |
| Disodium Lauryl Sulfosuccinate | 1,5 | 1,5 | 1,5 | 1,5 | 1,5 | 1,5 | 1,5 | 1,5 | 1,5 |
| Sodium Cocoamphoacetate | 3 | 3 | 3 | 3 | 3 | 3 | 3 | 3 | 3 |
| Chitosan | 0,25 | | | | | | | | |
| Hydrolyzed Keratin | | 0,15 | | | | 0,1 | | | |
| Hydrolyzed Wheat Protein | | | 0,32 | | | | | | |
| Hydroxypropyl Guar Hydroxypropyltrimonium Chloride | | | | 0,4 | | | | 0,1 | |
| Polyquarternium-11 | | | | | 0,15 | | | | |
| Polyquarternium-22 | | | | | | 0,4 | | | 0,28 |
| Polyquarternium-6 | 0,25 | | | | | | 0,4 | | |
| Polyquaternium-10 | | | | | | | | 0,3 | |
| Polyquaternium-28 | | | | | 0,15 | | | | 0,22 |
| Polyquaternium-37 (and) PPG-1 Trideceth-6; (E) - (and) Glycol Dicaprylate/Dicaprate | | | | | | | | | |
| Polyquaternium-7 | | | | | 0,1 | | | | |
| Polyquaternium-70 (and) Dipropylene Glycol | | | | | | | 0,1 | | |
| Quaternium-80 | | 0,35 | | | | | | | |
| Silicone Quaternium-22 | | | 0,18 | | | | | 0,1 | |
| Methoxy PEG/PPG-7/3 Aminopropyl Dimethicone | | | | 0,1 | | | | | |
| Polymer 50 M | | | | | | | | | |
| Polymer H 75 M | | | | | | | | | |
| Polymer quadrega | | | | | | | | | |
| Konservierung und Parfüm | q.s. | | | | | | | | |
| Wasser | ad 100,0% | | | | | | | | |
| | | | | | | | | | |

| Beispiel 3 cont. | **j** | k | **l** | m | n | **o** * | **p** | 4 * | r |
|---|---|---|---|---|---|---|---|---|---|
| RHEANCE^{®} One (Glycolipids) | 2,5 | 2,5 | 2,5 | 2,5 | 2,5 | 2,5 | 2,5 | 2,5 | 2,5 |
| Isostearamide MIPA; Glyceryl Laurate | 1 | 1 | 1 | 1 | 1 | 1 | 1 | 1 | 1 |
| Sorbitan Sesquicaprylate | 0,5 | 0,5 | 0,5 | 0,5 | 0,5 | 0,5 | 0,5 | 0,5 | 0,5 |
| Cocamidopropyl Betaine | 5 | 5 | 5 | 5 | 5 | 5 | 5 | 5 | 5 |
| Disodium Lauryl Sulfosuccinate | 1,5 | 1,5 | 1,5 | 1,5 | 1,5 | 1,5 | 1,5 | 1,5 | 1,5 |
| Sodium Cocoamphoacetate | 3 | 3 | 3 | 3 | 3 | 3 | 3 | 3 | 3 |
| Chitosan | | | | | | | | | |
| Hydrolyzed Keratin | | 0,12 | | | | | | | |
| Hydrolyzed Wheat Protein | | | | 0,25 | | | | | |
| Hydroxypropyl Guar Hydroxypropyltrimonium Chloride | | | | | | | | | |
| Polyquarternium-11 | | | | | | | | | 0,2 |
| Polyquarternium-22 | | | | | 0,15 | | | | |
| Polyquarternium-6 | | | | | | | | | |
| Polyquaternium-10 | | | | | | | 0,2 | | |
| Polyquaternium-28 | | | | | | | | | |
| Polyquaternium-37 (and) PPG-1 Trideceth-6; (E) - (and) Glycol Dicaprylate/Dicaprate | 0,19 | | | | | | | | |
| Polyquaternium-7 | | 0,28 | | | | | | | |
| Polyquaternium-70 (and) Dipropylene Glycol | | | 0,4 | | | | | | 0,2 |
| Quaternium-80 | 0,31 | | | 0,25 | | | | | |
| Silicone Quaternium-22 | | | | | 0,35 | | | | |
| Methoxy PEG/PPG-7/3 Aminopropyl Dimethicone | | | 0,1 | | | 0,5 | | | |
| Polymer 50 M | | | | | | | 0,3 | 0,2 | |
| Polymer H 75 M | | | | | | | | 0,2 | |
| Polymer quadrega | | | | | | | | 0,1 | 0,1 |
| Konservierung und Parfüm | q.s. | | | | | | | | |
| Wasser | ad 100,0% | | | | | | | | |

| Beispiel 4 | a * | b | c * | d | e | f | 9 | h | i |
|---|---|---|---|---|---|---|---|---|---|
| RHEANCE^{®} One (Glycolipids) | 3,00 | 3,00* | 3,00 | 3,00 | 3,00 | 3,00 | 3,00 | 3,00 | 3,00 |
| TEGO^{®} Betain F 50 (Cocamidopropyl Betaine) | 3,00 | 3,00 | 3,00 | 3,00 | 3,00 | 3,00 | 3,00 | 3,00 | 3,00 |
| Helianthus Annuus (Sunflower) Oil | 50,0 0 | 50,0 0 | 50,0 0 | 50,0 0 | 50,0 0 | 50,0 0 | 50,0 0 | 50,0 0 | 50,0 0 |
| ANTIL^{®} Soft SC (Sorbitan Sesquicaprylate) | 7,00 | 7,00 | 7,00 | 7,00 | 7,00 | 7,00 | 7,00 | 7,00 | 7,00 |
| VARISOFT^{®} EQ 100 (Bis-(Isostearoyl/Oleyl Isopropyl) Dimonium Methosulfate) | 1,00 | 1,00 | 1,00 | 1,00 | 1,00 | 1,00 | 1,00 | 1,00 | 1,00 |
| Chitosan | 0,20 | | | | | | | | |
| Hydrolyzed Keratin | | 0,30 | | | | | | | |
| Hydrolyzed Wheat Protein | | | 0,30 | | | | | | |
| Hydroxypropyl Guar Hydroxypropyltrimonium Chloride | | | | 0,20 | | | | | |
| Polyquarternium-11 | | | | | 0,10 | | | | |
| Polyquarternium-22 | | | | | | 0,2 | | | |
| Polyquarternium-6 | | | | | | | 0,4 | | |
| Polyquaternium-10 | | | | | | | | 0,7 | |
| Polyquaternium-28 | | | | | | | | | 0,25 |
| Polyquaternium-37 (and) PPG-1 Trideceth-6; (E) - (and) Glycol Dicaprylate/Dicaprate | | | | | | | | | |
| Polyquaternium-7 | | | | | | | | | |
| Polyquaternium-70 (and) Dipropylene Glycol | | | | | | | | | |
| Quaternium-80 | | | | | | | | | |
| Silicone Quaternium-22 | | | | | | | | | |
| Methoxy PEG/PPG-7/3 Aminopropyl Dimethicone | | | | | | | | | |
| Polymer 50 M | | | | | | | | | |
| Polymer H 75 M | | | | | | | | | |
| Polymer quadrega | | | | | | | | | |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |
| | | | | | | | | | |

| Beispiel 4 cont. | **j** | **k** | **l** | **m** | **n** | **o** | **p** * | **q** | **r** * |
|---|---|---|---|---|---|---|---|---|---|
| RHEANCE^{®} One (Glycolipids) | 3,00 | 3,00 | 3,00 | 3,00 | 3,00 | 3,00* | 3,00 | 3,00* | 3,00 |
| TEGO^{®} Betain F 50 (Cocamidopropyl Betaine) | 3,00 | 3,00 | 3,00 | 3,00 | 3,00 | 3,00 | 3,00 | 3,00 | 3,00 |
| Helianthus Annuus (Sunflower) Oil | 50,0 0 | 50,0 0 | 50,0 0 | 50,0 0 | 50,0 0 | 50,0 0 | 50,0 0 | 50,0 0 | 50,0 0 |
| ANTIL^{®} Soft SC (Sorbitan Sesquicaprylate) | 7,00 | 7,00 | 7,00 | 7,00 | 7,00 | 7,00 | 7,00 | 7,00 | 7,00 |
| VARISOFT^{®} EQ 100 (Bis-(Isostearoyl/Oleyl Isopropyl) Dimonium Methosulfate) | 1,00 | 1,00 | 1,00 | 1,00 | 1,00 | 1,00 | 1,00 | 1,00 | 1,00 |
| Chitosan | | | | | | | | | |
| Hydrolyzed Keratin | | | | | | | | | |
| Hydrolyzed Wheat Protein | | | | | | | | | |
| Hydroxypropyl Guar Hydroxypropyltrimonium Chloride | | | | | | | | | |
| Polyquarternium-11 | | | | | | | | | |
| Polyquarternium-22 | | | | | | | | | |
| Polyquarternium-6 | | | | | | | | | |
| Polyquaternium-10 | | | | | | | | | |
| Polyquaternium-28 | | | | | | | | | |
| Polyquaternium-37 (and) PPG-1 Trideceth-6; (E) - (and) Glycol Dicaprylate/Dicaprate | 0,3 | | | | | | | | |
| Polyquaternium-7 | | 0,4 | | | | | | | |
| Polyquaternium-70 (and) Dipropylene Glycol | | | 0,8 | | | | | | |
| Quaternium-80 | | | | 0,5 | | | | | |
| Silicone Quaternium-22 | | | | | 0,5 | | | | |
| Methoxy PEG/PPG-7/3 Aminopropyl Dimethicone | | | | | | 0,3 | | | |
| Polymer 50 M | | | | | | | 0,5 | | |
| Polymer H 75 M | | | | | | | | 0,5 | |
| Polymer quadrega | | | | | | | | | 0,5 |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |

| Beispiel 5 | **a** | **b** | **c** | **d** | **e** | **f** | **9** | **h** | **i** |
|---|---|---|---|---|---|---|---|---|---|
| RHEANCE^{®} One (Glycolipids) | 3* | 3* | 3* | 3 | 3 | 3 | 3 | 3 | 3 |
| Sodium Laureth Ether Sulfate | 3 | 3 | 3 | 3 | 3 | 3 | 6 | 6 | 6 |
| Cocamidopropyl Betaine | 3 | | | | | | 3 | | |
| Capryl/Capramidopropyl Betaine | | 3 | | | | | | 3 | |
| Coco-Betaine | | | 3 | | | | | | 3 |
| Sodium Cocoamphoacetate | | | | 3 | | | | | |
| Sodium Cocoamphopropionate | | | | | 3 | | | | |
| Disodium Cocoamphodiacetate | | | | | | 3 | | | |
| Chitosan | 0,5 | | | | | | | | |
| Hydrolyzed Keratin | | 0,5 | | | | | | | |
| Hydrolyzed Wheat Protein | | | 0,5 | | | | | | |
| Hydroxypropyl Guar Hydroxypropyltrimonium Chloride | | | | 0,5 | | | | | |
| Polyquarternium-11 | | | | | 0,5 | | | | |
| Polyquarternium-22 | | | | | | 0,5 | | | |
| Polyquarternium-6 | | | | | | | 0,5 | | |
| Polyquaternium-10 | | | | | | | | 0,5 | |
| Polyquaternium-28 | | | | | | | | | 0,5 |
| Polyquaternium-37 (and) PPG-1 Trideceth-6; (E) - (and) Glycol Dicaprylate/Dicaprate | | | | | | | | | |
| Polyquaternium-7 | | | | | | | | | |
| Polyquaternium-70 (and) Dipropylene Glycol | | | | | | | | | |
| Quaternium-80 | | | | | | | | | |
| Silicone Quaternium-22 | | | | | | | | | |
| Methoxy PEG/PPG-7/3 Aminopropyl Dimethicone | | | | | | | | | |
| Polymer 50 M | | | | | | | | | |
| Polymer H 75 M | | | | | | | | | |
| Polymer quadrega | | | | | | | | | |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |
| | | | | | | | | | |

| Beispiel 5 cont. | **j** | **k** | **l** | **m** | **n** | **o** * | **p *** | **q *** | **r *** |
|---|---|---|---|---|---|---|---|---|---|
| RHEANCE^{®} One (Glycolipids) | 3 | 3 | 3 | 6 | 6 | 6 | 6 | 6 | 6 |
| Sodium Laureth Ether Sulfate | 6 | 6 | 6 | 3 | 3 | 3 | 3 | 3 | 3 |
| Cocamidopropyl Betaine | | | | 3 | | | | | |
| Capryl/Capramidopropyl Betaine | | | | | 3 | | | | |
| Coco-Betaine | | | | | | 3 | | | |
| Sodium Cocoamphoacetate | 3 | | | | | | 3 | | |
| Sodium Cocoamphopropionate | | 3 | | | | | | 3 | |
| Disodium Cocoamphodiacetate | | | 3 | | | | | | 3 |
| Chitosan | | | | | | | | | |
| Hydrolyzed Keratin | | | | | | | | | |
| Hydrolyzed Wheat Protein | | | | | | | | | |
| Hydroxypropyl Guar Hydroxypropyltrimonium Chloride | | | | | | | | | |
| Polyquarternium-11 | | | | | | | | | |
| Polyquarternium-22 | | | | | | | | | |
| Polyquarternium-6 | | | | | | | | | |
| Polyquaternium-10 | | | | | | | | | |
| Polyquaternium-28 | | | | | | | | | |
| Polyquaternium-37 (and) PPG-1 Trideceth-6; (E) - (and) Glycol Dicaprylate/Dicaprate | 0,5 | | | | | | | | |
| Polyquaternium-7 | | 0,5 | | | | | | | |
| Polyquaternium-70 (and) Dipropylene Glycol | | | 0,5 | | | | | | |
| Quaternium-80 | | | | 0,5 | | | | | |
| Silicone Quaternium-22 | | | | | 0,5 | | | | |
| Methoxy PEG/PPG-7/3 Aminopropyl Dimethicone | | | | | | 0,5 | | | |
| Polymer 50 M | | | | | | | 0,5 | | |
| Polymer H 75 M | | | | | | | | 0,5 | |
| Polymer quadrega | | | | | | | | | 0,5 |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |

| Beispiel 6 | **a** * | **b** | **c** | **d** | **e** | **f** | **9** | **h** | **i** |
|---|---|---|---|---|---|---|---|---|---|
| RHEANCE^{®} One (Glycolipids) | 3 | 8,2 | 1,9 | 2,9 | 0,5 | 3 | 6 | 6 | 3 |
| Sodium Laureth Ether Sulfate | 1,5 | | | | 2,5 | | | | 1 |
| Ammonium Laureth Sulfate | | | 0,5 | | 2,1 | 1 | | 1 | |
| Sodium Cocoyl Glutamate | 3 | 1 | | 2,5 | 0,1 | | 1 | | |
| Sodium Cocoyl Glycinate | | | | 2,5 | | 1 | | | 3 |
| Sodium Cocoyl Sarcosinate | | | 3 | | 1 | 1 | | 2 | |
| Sodium Laureth Sulfate | 1 | | | | | | | | |
| Sodium Lauroyl Methyl Isethionate | | | 1 | | 0,5 | | 3 | | 2 |
| Sodium Lauryl Sulfate | | 1 | | 2,5 | | 1 | | | |
| Sodium/Disodium Cocoyl Glutamate | 1 | | | | | | | 3 | |
| Stearic Acid | 0,2 | | | 0,1 | | | | | |
| Olea Europaea (Olive) Fruit Oil | | | | 0,1 | | | | | |
| Panthenol | | | 0,2 | | 0,1 | | | | |
| Persea Gratissima (Avocado) Oil | | | 0,1 | | | | | | 0,25 |
| Salicylic Acid | 0,2 | | | | | | 0,2 | | |
| Zinc Pyrithione | | 0,2 | | | | | | | |
| Tetrasodium EDTA | | | | 0,1 | | 0,05 | | 0,1 | |
| Octopirox | | 0,2 | | | 0,25 | | | | |
| Acrylates / C10-30 Alkyl Acrylate Crosspolymer | | | | | | | | 0,5 | |
| Glycerin | | 1 | | | 1 | | 1 | | |
| Glycine Soja (Soybean) Oil | 0,1 | | | 0,1 | | | | 0,1 | |
| Glycol Distearate | | | | | | | | | |
| Dimethicone | | | | | | | | | |
| Creatine | | | | | | 0,1 | | | 0,2 |
| Benzophenone-4 | | | | | | | | | |
| Benzyl Alcohol | | | | | | | | | |
| Butyrospermum Parkii Butter Extract | | | | | | | | | |
| Caffeine | | | | | | | | | 0,25 |
| Cellulose | 0,1 | | | | 0,15 | | | | |
| Olea Europaea (Olive) Fruit Oil | | 0,1 | 0,1 | | | | | | |
| Chitosan | 0,2 | | | | | | | | |
| Hydrolyzed Keratin | | 0,2 | | | | | | | |
| Hydrolyzed Wheat Protein | | | 0,2 | | | | | | |
| Hydroxypropyl Guar Hydroxypropyltrimonium Chloride | | | | 0,2 | | | | | |
| Polyquarternium-11 | | | | | 0,2 | | | | |
| Polyquarternium-22 | | | | | | 0,2 | | | |
| Polyquarternium-6 | | | | | | | 0,2 | | |
| Polyquaternium-10 | | | | | | | | 0,2 | |
| Polyquaternium-28 | | | | | | | | | 0,2 |
| Polyquaternium-37 (and) PPG-1 Trideceth-6; (E) - (and) Glycol Dicaprylate/Dicaprate | | | | | | | | | |
| Polyquaternium-7 | | | | | | | | | |
| Polyquaternium-70 (and) Dipropylene Glycol | | | | | | | | | |
| Quaternium-80 | | | | | | | | | |
| Silicone Quaternium-22 | | | | | | | | | |
| Methoxy PEG/PPG-7/3 Aminopropyl Dimethicone | | | | | | | | | |
| Polymer 50 M | | | | | | | | | |
| Polymer H 75 M | | | | | | | | | |
| Polymer quadrega | | | | | | | | | |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |
| | | | | | | | | | |

| Beispiel 6 cont. | **j** | **k** | l | **m** | **n** | **o** * | **p** * | **q** * | **r** * |
|---|---|---|---|---|---|---|---|---|---|
| RHEANCE^{®} One (Glycolipids) | 3,5 | 2 | 1 | 4,5 | 3,9 | 4,6 | 5,5 | 2,5 | 2 |
| Sodium Laureth Ether Sulfate | | | | | 1 | | | | |
| Ammonium Laureth Sulfate | | | | 1 | | | | | 1 |
| Sodium Cocoyl Glutamate | | | 1 | | | 2 | | 1 | |
| Sodium Cocoyl Glycinate | | 1 | | | | | 1 | | |
| Sodium Cocoyl Sarcosinate | 1 | | | | | 1 | | | |
| Sodium Laureth Sulfate | | 5 | | 4 | | | | | 4,5 |
| Sodium Lauroyl Methyl Isethionate | | | | | | 1,8 | | | |
| Sodium Lauryl Sulfate | | | | | | | | 3,5 | |
| Sodium/Disodium Cocoyl Glutamate | | | | 2,5 | | | 2,5 | | |
| Stearic Acid | 0,1 | | 0,4 | | 0,2 | | | 0,1 | 0,2 |
| Olea Europaea (Olive) Fruit Oil | | | | | | 0,3 | | | |
| Panthenol | | 0,3 | | | | | 0,3 | | |
| Persea Gratissima (Avocado) Oil | | | | | | | | 0,2 | |
| Salicylic Acid | | | | | | 0,3 | | 0,1 | |
| Zinc Pyrithione | 0,2 | | | 0,2 | | 0,2 | | | |
| Tetrasodium EDTA | | | | | | | | | 0,1 |
| Octopirox | | | | 0,1 | | | | 0,1 | |
| Acrylates / C10-30 Alkyl Acrylate Crosspolymer | | | | | | | | | |
| Glycerin | 1 | 3 | | 2 | | 5 | | 3 | 1 |
| Glycine Soja (Soybean) Oil | | | 0,1 | | | | | | |
| Glycol Distearate | | | | | | | | | |
| Dimethicone | | | | 0,3 | | | | | |
| Creatine | | | | | | | | | |
| Benzophenone-4 | | | | | | | | | |
| Benzyl Alcohol | | | 0,1 | | | | | | 0,2 |
| Butyrospermum Parkii Butter Extract | | | | | | | | 0,2 | |
| Caffeine | | | | 0,1 | | | | | |
| Cellulose | | | | | | | | | |
| Olea Europaea (Olive) Fruit Oil | | | | | | | | | |
| Chitosan | | | | | | | | | |
| Hydrolyzed Keratin | | | | | | | | | |
| Hydrolyzed Wheat Protein | | | | | | | | | |
| Hydroxypropyl Guar Hydroxypropyltrimonium Chloride | | | | | | | | | |
| Polyquarternium-11 | | | | | | | | | |
| Polyquarternium-22 | | | | | | | | | |
| Polyquarternium-6 | | | | | | | | | |
| Polyquaternium-10 | | | | | | | | | |
| Polyquaternium-28 | | | | | | | | | |
| Polyquaternium-37 (and) PPG-1 Trideceth-6; (E) - (and) Glycol Dicaprylate/Dicaprate | 0,2 | | | | | | | | |
| Polyquaternium-7 | | 0,2 | | | | | | | |
| Polyquaternium-70 (and) Dipropylene Glycol | | | 0,2 | | | | | | |
| Quaternium-80 | | | | 0,2 | | | | | |
| Silicone Quaternium-22 | | | | | 0,2 | | | | |
| Methoxy PEG/PPG-7/3 Aminopropyl Dimethicone | | | | | | 0,2 | | | |
| Polymer 50 M | | | | | | | 0,2 | | |
| Polymer H 75 M | | | | | | | | 0,2 | |
| Polymer quadrega | | | | | | | | | 0,2 |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |

| Beispiel 7 | **a** | **b** | **c** | **d** | **e** | **f** | **9** | **h** | **i** |
|---|---|---|---|---|---|---|---|---|---|
| RHEANCE^{®} One (Glycolipids) | 7,5 | 5,5 | 5 | 2,8 | 3,9 | 2,9 | 4,5 | 6 | 8 |
| Coco-Glucoside | 10 | 2 | 1 | 1 | | 1 | 6 | 1,5 | 1 |
| Lauryl Glucoside | | 5 | 1 | 5 | 5 | 3 | 2,5 | 1,8 | 1 |
| Decyl Glucoside | | 3 | 1 | 2 | 3 | 2 | 2,5 | 3 | 1 |
| Xanthan Gum | | | 0,5 | | | 0,5 | | | |
| Chitosan | | | | | | | | | |
| Hydrolyzed Keratin | | 0,1 | | | | | | | |
| Hydrolyzed Wheat Protein | | | | | | | | | 0,25 |
| Hydroxypropyl Guar Hydroxypropyltrimonium Chloride | | | | | | | | | |
| Polyquarternium-11 | | | | | | | 0,3 | | |
| Polyquarternium-22 | | | | | | | | | |
| Polyquarternium-6 | | | | | 0,2 | | | 0,1 | |
| Polyquaternium-10 | | | | | | | | | |
| Polyquaternium-28 | 0,1 | 0,3 | 0,1 | | | 0,1 | | | |
| Polyquaternium-37 (and) PPG-1 Trideceth-6; (E) - (and) Glycol Dicaprylate/Dicaprate | 0,5 | | | | | | | | |
| Polyquaternium-7 | | | | 1,2 | | | | | |
| Polyquaternium-70 (and) Dipropylene Glycol | | | | | | | | | |
| Quaternium-80 | | 0,1 | | | 0,1 | 0,1 | | | 0,1 |
| Silicone Quaternium-22 | | | | | | 0,1 | | | |
| Methoxy PEG/PPG-7/3 Aminopropyl Dimethicone | | | | | | | | | |
| Polymer 50 M | | | | | | | | 0,3 | |
| Polymer H 75 M | | | | | | | 0,4 | | |
| Polymer quadrega | | | | | | 0,2 | | | |
| pH-Wert | 5 | 5 | 4,8 | 5 | 5,9 | 5 | 5,9 | 4,9 | 4,8 |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |
| | | | | | | | | | |

| Beispiel 7 cont. | **j** * | **k** * | **l *** | **m** | **n** | **o** | **p** | **q** | **r** |
|---|---|---|---|---|---|---|---|---|---|
| RHEANCE^{®} One (Glycolipids) | 5 | 2 | 1,1 | 1,1 | 1,7 | 1,5 | 5,9 | 6,9 | 10 |
| Coco-Glucoside | | | 2 | | 3 | | | 2 | 1 |
| Lauryl Glucoside | | 5 | | 2 | | 3 | 2 | | 1 |
| Decyl Glucoside | | | 5 | 5 | 3 | 5 | 2 | 2 | |
| Xanthan Gum | 0,9 | 0,8 | | 0,9 | | | 0,3 | | |
| Chitosan | | 0,29 | | | 0,1 | | | | |
| Hydrolyzed Keratin | | | | | | | | | |
| Hydrolyzed Wheat Protein | | | 0,25 | | | | | | |
| Hydroxypropyl Guar Hydroxypropyltrimonium Chloride | | | | | | 0,23 | | | |
| Polyquarternium-11 | | | | | | | | | |
| Polyquarternium-22 | | | | | | | | 0,29 | |
| Polyquarternium-6 | | | | | | | | | |
| Polyquaternium-10 | | | | | | | 0,45 | | |
| Polyquaternium-28 | | | | | | 0,2 | | | |
| Polyquaternium-37 (and) PPG-1 Trideceth-6; (E) - (and) Glycol Dicaprylate/Dicaprate | | | | | | | | | |
| Polyquaternium-7 | | | | | | | | | |
| Polyquaternium-70 (and) Dipropylene Glycol | | | | | | | | | 0,39 |
| Quaternium-80 | | | | 0,2 | | | | | |
| Silicone Quaternium-22 | | | | 0,15 | | | | | |
| Methoxy PEG/PPG-7/3 Aminopropyl Dimethicone | 0,25 | | | | | | | | |
| Polymer 50 M | | | | | | | | | |
| Polymer H 75 M | | | | | 0,3 | | | | |
| Polymer quadrega | | | | | | | | 0,2 | |
| pH-Wert | 5,2 | 6,3 | 5,3 | 4,9 | 5 | 6,5 | 4,5 | 5,2 | 4,7 |
| Konservierung und Parfüm | q.s. | q.s. | q.s. | q.s. | q.s. | q.s. | q.s. | q.s. | q.s. |
| Wasser | auf 100 | auf 100 | auf 100 | auf 100 | auf 100 | auf 100 | auf 100 | auf 100 | auf 100 |

| Beispiel 8 | **a** | **b** | **c** | **d** | **e** | **f** | **9** | **h** | **i** |
|---|---|---|---|---|---|---|---|---|---|
| RHEANCE^{®} One (Glycolipids) | 8 | 9 | 10 | 5 | 8 | 4 | 3 | 9 | 6 |
| Palmitamidopropyltrimonium Chloride | 0,5 | | | | | 0,5 | 0,3 | | 0,1 |
| Cocamide DEA | 0,5 | | | 0,5 | | | | | |
| Cocamide MEA | | 0,5 | | | 0,5 | | | | |
| Glycol Distearate | | | | | | 0,3 | | | |
| Disodium PEG-4 Cocamido MIPA-Sulfosuccianate | | | | | | 0,5 | | | |
| Dicaprylylether | | | | | | | | | |
| Isostearamide MIPA | | | | | | 0,2 | | | |
| Oleyl Erucate | | | | | | | | | 1 |
| PEG-200 Hydrogenated Glyceryl Palmate; PEG-7 Glyceryl Cocoate | | | | | | | | 1 | |
| PEG-40 Hydrogenated Castor Oil | | | | | 1 | | | | |
| Polyglyceryl-4 Caprate | 3 | | | | | | | | |
| Propylene Glycol; PEG-55 Propylene Glycol Oleate | | | | | | | | | |
| Prunus Amygdalus Dulcis (Sweet Almond) Oil | 2 | | | | 1 | | 0,5 | | |
| Sodium C14-16 Olefin Sulfonate | | 3 | | 5 | | 5 | 10 | 1 | |
| Sodium Chloride | | | 2 | | | | | | |
| Chitosan | | | | | | | | | |
| Hydrolyzed Keratin | | | | | | | | | |
| Hydrolyzed Wheat Protein | | 0,23 | | | | | | | 0,25 |
| Hydroxypropyl Guar Hydroxypropyltrimonium Chloride | 0,36 | | | | | | | 0,9 | |
| Polyquarternium-11 | | | | | | | 0,2 | | |
| Polyquarternium-22 | 0,28 | | | 0,4 | | | | | |
| Polyquarternium-6 | | | | | | | | 0,1 | |
| Polyquaternium-10 | | | | | 0,4 | | | 0,3 | |
| Polyquaternium-28 | 0,22 | | | | | | | | |
| Polyquaternium-37 (and) PPG-1 Trideceth-6; (E) - (and) Glycol Dicaprylate/Dicaprate | | | | | | | | | |
| Polyquaternium-7 | | 0,28 | | | | | | | |
| Polyquaternium-70 (and) Dipropylene Glycol | | | 0,1 | | | | 0,2 | | |
| Quaternium-80 | | | | | | | | | 0,25 |
| Silicone Quaternium-22 | | | | | | 0,2 | | | |
| Methoxy PEG/PPG-7/3 Aminopropyl Dimethicone | | | | | | | | | |
| Polymer 50 M | | | | | 0,3 | 0,2 | | | |
| Polymer H 75 M | | 0,12 | | 0,1 | | | | | |
| Polymer quadrega | | | | | | 0,1 | 0,9 | | |
| | | | | | | | | | |

| Beispiel 8 cont. | **j** | **k** | **l** | **m** | **n** | **o** | **p** | **q** | **r** |
|---|---|---|---|---|---|---|---|---|---|
| RHEANCE^{®} One (Glycolipids) | 3,5 | 11,5 | 15 | 10 | 11 | 9,5 | 8,5 | 2,5 | 5,5 |
| Palmitamidopropyltrimonium Chloride | | 1,5 | | | | 1 | | | |
| Cocamide DEA | | | | | | | | | |
| Cocamide MEA | | | | | | | | | |
| Glycol Distearate | | | | | | | | | |
| Disodium PEG-4 Cocamido MIPA-Sulfosuccianate | 0,5 | | | | | | | | |
| Dicaprylylether | | | 0,2 | 0,2 | | | | 0,5 | |
| Isostearamide MIPA | | 1 | | | | | | | |
| Oleyl Erucate | | | | | | | | | |
| PEG-200 Hydrogenated Glyceryl Palmate; PEG-7 Glyceryl Cocoate | | | | | | | | | |
| PEG-40 Hydrogenated Castor Oil | | | | | | | | 5 | 3 |
| Polyglyceryl-4 Caprate | | | | 1 | | | | | |
| Propylene Glycol; PEG-55 Propylene Glycol Oleate | | | | | 0,5 | | | | |
| Prunus Amygdalus Dulcis (Sweet Almond) Oil | | | | | | 0,1 | 0,1 | 0,1 | 0,1 |
| Sodium C14-16 Olefin Sulfonate | 10 | | | | | | | 12 | 10 |
| Sodium Chloride | 1 | | 1,5 | 1 | | 0,25 | | | |
| Chitosan | | | | | | | 0,25 | | |
| Hydrolyzed Keratin | | | | | | | 0,25 | | |
| Hydrolyzed Wheat Protein | | 0,32 | | | | | | | |
| Hydroxypropyl Guar Hydroxypropyltrimonium Chloride | | | | 0,4 | | | | | |
| Polyquarternium-11 | | | | | | | | | 0,25 |
| Polyquarternium-22 | | | | | 0,75 | | | | |
| Polyquarternium-6 | | 0,18 | | | 0,35 | | | | |
| Polyquaternium-10 | | | | | | | | | |
| Polyquaternium-28 | | | | | | | | | 0,55 |
| Polyquaternium-37 (and) PPG-1 Trideceth-6; (E) - (and) Glycol Dicaprylate/Dicaprate | 0,39 | | | | | | | | |
| Polyquaternium-7 | | | | | | | | | 0,1 |
| Polyquaternium-70 (and) Dipropylene Glycol | | | 0,4 | | | | 0,3 | | |
| Quaternium-80 | 0,31 | | | | | | | 0,35 | |
| Silicone Quaternium-22 | | | | | 0,5 | | | | |
| Methoxy PEG/PPG-7/3 Aminopropyl Dimethicone | | | 0,55 | 0,5 | | 0,5 | | | |
| Polymer 50 M | | | | | | | | | |
| Polymer H 75 M | | | | | | | | 0,15 | |
| Polymer quadrega | | | | | | | | | |

## Patentansprüche

1. Verwendung von Rhamnolipiden zur Deposition mindestens einer Substanz aus einem Medium auf einer Oberfläche, **dadurch gekennzeichnet, dass** die Substanz ausgewählt ist aus der Gruppe umfassend Guar-Quats, Siloxan-Quats und Cellulose-Quats und der pH-Wert des Mediums bei 25 °C in einem Bereich von 3,5 bis 9,0 liegt.

2. Verwendung nach Anspruch 1, **dadurch gekennzeichnet, dass** das Medium ausgewählt ist aus der Gruppe umfassend Tensidmischungen, Emulsionen und Mikroemulsionen, insbesondere O/W-Emulsionen, W/O-Emulsionen, Wasser-in-Silicon-Emulsionen, Silicon-in-Wasser-Emulsionen, O/W-Mikroemulsionen, W/O-Mikroemulsionen, Wasser-in-Silicon-Mikroemulsionen, Silicon-in-Wasser-Mikroemulsionen sowie Dispersionen.

3. Verwendung gemäß mindestens einem der vorherigen Ansprüche, **dadurch gekennzeichnet, dass** das Medium ausgewählt ist aus der Gruppe umfassend kosmetische Formulierungen.

4. Verwendung gemäß mindestens einem der vorherigen Ansprüche, **dadurch gekennzeichnet, dass** die Rhamnolipide in dem Medium in einer Konzentration von 0,1 Gew.-% bis 30 Gew.-%, bevorzugt von 0,5 Gew.-% bis 25 Gew.-%, besonders bevorzugt von 1,0 Gew.-% bis 12 Gew.-%, enthalten sind, wobei sich die Gewichtsprozente auf das Gesamtmedium beziehen.

5. Verwendung gemäß mindestens einem der vorherigen Ansprüche, **dadurch gekennzeichnet, dass** die Substanz in dem Medium in einer Konzentration von 0,05 Gew.-% bis 5 Gew.-%, bevorzugt von 0,1 Gew.-% bis 3 Gew.-%, besonders bevorzugt von 0,15 Gew.-% bis 1,5 Gew.-%, enthalten ist, wobei sich die Gewichtsprozente auf das Gesamtmedium beziehen.

6. Verwendung gemäß mindestens einem der vorherigen Ansprüche, **dadurch gekennzeichnet, dass** das Gewichtsverhältnis von allen verwendeten di-Rhamnolipiden zu allen verwendeten mono-Rhamnolipiden größer 51:49, insbesondere größer 91:9, bevorzugt größer 97:3, besonders bevorzugt größer 98:2, ist.

7. Verwendung gemäß mindestens einem der vorherigen Ansprüche, **dadurch gekennzeichnet, dass** der pH-Wert des Mediums bei 25 °C in einem Bereich von 3,8 bis 7,0 besonders bevorzugt von 4,4 bis 6,6 liegt.

8. Nicht-therapeutische Verwendung gemäß mindestens einem der vorherigen Ansprüche, **dadurch gekennzeichnet, dass** die Oberfläche ausgewählt ist aus Haut und Haaren.

## Claims

1. Use of rhamnolipids for the deposition of at least one substance from a medium onto a surface, **characterized in that** the substance is selected from the group comprising guar quats, siloxane quats and cellulose quats and the pH of the medium at 25°C is in a range from 3.5 to 9.0.

2. Use according to Claim 1, **characterized in that** the medium is selected from the group comprising surfactant mixtures, emulsions and microemulsions, in particular O/W emulsions, W/O emulsions, water-in-silicone emulsions, silicone-in-water emulsions, O/W microemulsions, W/O microemulsions, water-in-silicone microemulsions, silicone-in-water microemulsions, and dispersions.

3. Use according to at least one of the preceding claims, **characterized in that** the medium is selected from the group comprising cosmetic formulations.

4. Use according to at least one of the preceding claims, **characterized in that** the rhamnolipids are present in the medium at a concentration of 0.1% by weight to 30% by weight, preferably of 0.5% by weight to 25% by weight, particularly preferably of 1.0% by weight to 12% by weight, wherein the percentages by weight relate to the total medium.

5. Use according to at least one of the preceding claims, **characterized in that** the substance is present in the medium at a concentration of 0.05% by weight to 5% by weight, preferably of 0.1% by weight to 3% by weight, particularly preferably of 0.15% by weight to 1.5% by weight, wherein the percentages by weight relate to the total medium.

6. Use according to at least one of the preceding claims, **characterized in that** the weight ratio of all di-rhamnolipids used to all mono-rhamnolipids used is greater than 51:49, in particular greater than 91:9, preferably greater than 97:3, particularly preferably greater than 98:2.

7. Use according to at least one of the preceding claims, **characterized in that** the pH of the medium at 25°C is in a range from 3.8 to 7.0, particularly preferably from 4.4 to 6.6.

8. Non-therapeutic use according to at least one of the preceding claims, **characterized in that** the surface is selected from skin and hair.

## Revendications

1. Utilisation de rhamnolipides pour le dépôt d'au moins une substance d'un milieu sur une surface, **caractérisée en ce que** la substance est choisie dans le groupe comprenant les guar-quats, les siloxane-quats et les cellulose-quats, et le pH du milieu à 25 °C est compris dans la plage de 3,5 à 9,0.

2. Utilisation selon la revendication 1, **caractérisée en ce que** le milieu est choisi dans le groupe comprenant les mélanges de tensioactifs, les émulsions et les microémulsions, en particulier les émulsions de type aqueux, les émulsions de type huileux, les émulsions eau-dans-silicone, les émulsions silicone-dans-eau, les microémulsions de type aqueux, les microémulsions de type huileux, les microémulsions eau-dans-silicone, les microémulsions silicone-dans-eau, ainsi que les dispersions.

3. Utilisation selon au moins l'une des revendications précédentes, **caractérisée en ce que** le milieu est choisi dans le groupe comprenant des formulations cosmétiques.

4. Utilisation selon au moins l'une des revendications précédentes, **caractérisée en ce que** les rhamnolipides sont présents dans le milieu selon une concentration de 0,1 % en poids à 30 % en poids, de préférence de 0,5 % en poids à 25 % en poids, d'une manière particulièrement préférée de 1,0 % en poids à 12 % en poids, les pourcentages en poids étant rapportés au milieu total.

5. Utilisation selon au moins l'une des revendications précédentes, **caractérisée en ce que** la substance est présente dans le milieu selon une concentration de 0,05 % en poids à 5 % en poids, de préférence de 0,1 % en poids à 3 % en poids, d'une manière particulièrement préférée de 0,15 % en poids à 1,5 % en poids, les pourcentages en poids étant rapportés au milieu total.

6. Utilisation selon au moins l'une des revendications précédentes, **caractérisée en ce que** le rapport en poids de tous les di-rhamnolipides utilisés à tous les mono-rhamnolipides utilisés est supérieur à 51:49, en particulier supérieur à 91:9, de préférence supérieur à 97:3, d'une manière particulièrement préférée supérieur à 98:2.

7. Utilisation selon au moins l'une des revendications précédentes, **caractérisée en ce que** le pH du milieu à 25 °C est compris dans la plage de 3,8 à 7,0, d'une manière particulièrement préférée de 4,4 à 6, 6.

8. Utilisation non thérapeutique selon au moins l'une des revendications précédentes, **caractérisée en ce que** la surface est choisie parmi la peau et les cheveux.
